# EUROPÄISCHE PATENTANMELDUNG

(11) **EP 2 524 673 A1**
(43) Veröffentlichungstag der Anmeldung: **21.11.2012**
(21) Anmeldenummer: 12168160.5
(22) Anmeldetag: 16.05.2012
(51) Int. Cl.: A61F 5/01

(54) **Stützkorpus für Knie-Orthesen bzw. Kniegelenk-Stützen**

(30) Priorität: 19.05.2011 AT 7192011
(71) Anmelder: Wayd, Kurt, 1020 Wien (AT)
(72) Erfinder: Wayd, Kurt, 1020 Wien (AT); Grafinger, Josef, 1160 Wien (AT)
(74) Vertreter: Pinter, Rudolf

(57) **Zusammenfassung**

Stützkorpus (1) für Knie-Orthesen bzw. Kniegelenk-Stützen, in Form einer sich entlang des Unterschenkels erstreckenden und diesen teilweise umfassenden Schiene (2), die am oberen Ende mit einem Orthesen-Gelenk bzw. einem Gelenk der Kniegelenk-Stütze verbunden und am unteren Ende mit einem zumindest die Ferse aufnehmenden Fußteil (3) versehen ist.

Um trotz optimaler Stützwirkung die Beweglichkeit des Beins möglichst wenig zu behindern, sind am oberen Ende der Schiene zwei seitlich des Beins angeordnete Anbindungsstellen (4) für die Knie-Orthese bzw. die Kniegelenk-Stützen vorgesehen, die in einen am Schienbein entlang des Beins nach unten verlaufenden Abschnitt (2) übergehen, welcher seinerseits oberhalb des Knöchelbereiches wieder in zwei seitlich des Beins verlaufende Verbindungsteile (6) zum Fußteil (3) übergeht.

## Beschreibung

Die Erfindung betrifft einen Stützkorpus für Knie-Orthesen bzw. Kniegelenk-Stützen, in Form einer sich entlang des Unterschenkels erstreckenden und diesen teilweise umfassenden Schiene, die am oberen Ende mit einem Orthesen-Gelenk bzw. einem Gelenk der Kniegelenk-Stütze verbunden und am unteren Ende mit einem zumindest die Ferse aufnehmenden Fußteil versehen ist.

Derartige Konstruktionen sind in vielfachen Ausführungsformen bekannt. Beispielsweise ist in der CA 2543217 A eine Beinorthese geoffenbart, die eine sich entlang des Oberschenkels und des Unterschenkels bis zu einem Fußteil erstreckende Stütze bietet. Die Stützstruktur kann am Oberschenkel und am Unterschenkel durch Bandagen fixiert werden, welche diese Beinabschnitte gänzlich umfassen, was aber zu unterschiedlicher Festigkeit der Fixierung je nach dem Grad der Bewegung der Muskelanspannung im Bein führt.

Die Aufgabe der vorliegenden Erfindung ist eine Konstruktion, bei welcher trotz optimaler Stützwirkung die Beweglichkeit des Beins möglichst wenig behindert ist.

Zur Lösung dieser Aufgabe ist erfindungsgemäß vorgesehen, dass am oberen Ende der Schiene zwei seitlich des Beins angeordnete Anbindungsstellen für die Knie-Orthese bzw. die Kniegelenk-Stützen vorgesehen sind, die in einen am Schienbein entlang des Beins nach unten verlaufenden Abschnitt übergehen, welcher seinerseits oberhalb des Knöchelbereiches wieder in zwei seitlich des Beins verlaufende Verbindungsteile zum Fußteil übergeht.

Gemäß einer vorteilhaften Ausführungsform der Erfindung ist vorgesehen, dass der Schienbein-Abschnitt um das Bein herum gekrümmt oder geknickt ausgeführt ist, so dass das Bein über einen Teil seines Umfanges vom Abschnitt abgedeckt ist.

Eine andere Ausführungsform ist zusätzlich **dadurch gekennzeichnet, dass** in den Verbindungsteilen unterhalb der Knöchelhöhe jeweils ein Gelenk zur schwenkbaren Verbindung zwischen Schienbein-Abschnitt und Fußteil vorgesehen ist.

Vorteilhafterweise ist dabei vorgesehen, dass die Achse der Gelenke in den Verbindungsteilen an der Fußaußenseite weiter in Richtung Ferse liegt als an der Fußinnenseite.

Erfindungsgemäß kann ein Stützkorpus auch weiters **dadurch gekennzeichnet sein, dass** das Orthesen-Gelenk bzw. das Gelenk der Kniegelenk-Stütze und die Schiene über zwei monozentrische Gelenke miteinander verbunden sind, deren Achsen im Wesentlichen in der Ebene der Beugebewegung des Kniegelenks orientiert sind.

Eine wieder andere Ausführungsform der Erfindung sieht vor, dass das Orthesen-Gelenk bzw. das Gelenk der Kniegelenk-Stütze und die Schiene über zwei Gelenks-Elemente miteinander verbunden sind, die eine Rotationsbewegung zwischen Stützkorpus und Orthesen-Gelenk bzw. Gelenk der Kniegelenk-Stütze um die Achse des Unterschenkels ermöglichen.

Dabei ist eine vorteilhafte Variante **dadurch gekennzeichnet, dass** jedes Gelenks-Element aus zwei ineinander bzw. aneinander entlang einer Kreissegmentbahn geführten Teilstücken besteht, wobei die Kreissegmente beider Gelenks-Elemente einem Kreis mit Mittelpunkt im zentralen Bereich des Beins zugehören.

Zweckmäßigerweise ist eine weitere Ausführungsform der Erfindung **dadurch gekennzeichnet, dass** eine Einrichtung zur Abstandsverstellung zwischen Orthesen-Gelenk bzw. Gelenk der Kniegelenk-Stütze und Schienbein-Schiene an zumindest einer der Anbindungsstellen, vorzugsweise an beiden Anbindungsstellen, vorgesehen ist.

Vorzugsweise sind dabei die monozentrischen Gelenke oberhalb der Gelenks-Elemente für die Rotationsbewegung angeordnet.

Auch die bzw. jede Einrichtung zur Abstandsverstellung kann unterhalb jedes vorgesehen Gelenks bzw. Gelenk-Elementes angeordnet sein.

In der nachfolgenden Beschreibung soll die Erfindung, auch anhand eines konkreten Ausführungsbeispiels, näher erläutert werden, wobei diese Ausführungen jedoch keinerlei Einschränkung darstellen.

Dabei zeigt die Zeichnungsfigur eine Ansicht einer Ausführungsform eines erfindungsgemäßen Stützkorpus.

Der Stützkorpus 1 umfasst erfindungsgemäß eine sich entlang des Unterschenkels erstreckende und diesen teilweise umfassende Schiene 2, die am oberen Ende mit einem Orthesen-Gelenk bzw. einem Gelenk einer Kniegelenk-Stütze (nicht dargestellt) verbunden ist. Am unteren Ende ist ein zumindest die Ferse aufnehmender Fußteil 3 beweglich befestigt.

Um das Gewicht des Trägers und die Belastung während der Benutzung optimal abzustützen, sind am oberen Ende der Schiene 2 zwei seitlich des Beins angeordnete Anbindungsstellen 4 für die Knie-Orthese bzw. die Kniegelenk-Stützen vorgesehen. Von diesen Anbindungsstellen 4 verlaufen zwei seitliche Äste 5 des Stützkorpus 1 seitlich entlang des Beins nach unten und nach vorne zu und gehen in den am Schienbein entlang des Beins nach unten verlaufenden Abschnitt der Schiene 2 über, welcher vorteilhafterweise um das Bein herum gekrümmt oder geknickt ausgeführt ist, so dass das Bein vorne über einen Teil seines Umfanges von der Schiene 2 abgedeckt ist, der hintere Bereich des Beins aber frei bleibt. Die Schiene 2 liegt vorne mit ihrer vorzugsweise etwas gepolsterten Innenseite unmittelbar am Schienbeinknochen an. Vorzugsweise ist dieser Abschnitt einstückig mit den zwei seitlichen Ästen 5 ausgeführt. Dadurch kann bei leichter Bauweise die notwendige Steifigkeit gewährleistet werden.

Oberhalb des Knöchelbereiches teilt sich die Schiene 2 ebenfalls in zwei seitlich des Beins verlaufende Verbindungsteile 6 zum Fußteil 3. Die Verbindungsteile 6 sind vorteilhafterweise anatomisch dem Knöchelgelenk entsprechend nach außen hin ausgebuchtet.

In den Verbindungsteilen 6 ist unterhalb der Knöchelhöhe jeweils ein Gelenk 7 vorgesehen, über welches der Fußteil 3 schwenkbar am Stützkorpus 1 befestigt ist. Die Gelenke 7 kommen damit etwa 30 bis 70 mm, vorzugsweise etwa 55mm, oberhalb der Sohle des Fußteils 3 zu liegen. Weiters ist vorgesehen, dass die Achse der Gelenke 7 derart orientiert sind, dass sie an der Fußaußenseite weiter in Richtung Ferse versetzt ist als an der Fußinnenseite. Vom hinteren Ende des Fußteils 3, an welchem die Ferse anliegt, ist die Achse der Gelenke an der Fußaußenseite etwa 40 bis 70 mm, vorzugsweise etwa 55mm, nach vorne versetzt und an der Fußinnenseite noch weitere 10 bis 20 mm, vorzugsweise 15 mm, nach vorne versetzt.

Das Orthesen-Gelenk bzw. das Gelenk der Kniegelenk-Stütze und die seitlichen Äste 5 können über zwei monozentrische Gelenke 8 miteinander verbunden sein, deren Achsen im Wesentlichen in der Ebene der Beugebewegung des Kniegelenks orientiert sind. Zusätzlich oder auch alternativ dazu können zwischen Orthesen-Gelenk bzw. Gelenk der Kniegelenk-Stütze und den seitlichen Ästen 5 des Stützkorpus 1 über zwei Gelenks-Elemente 9 miteinander verbunden sein, die eine Rotationsbewegung zwischen Stützkorpus 1 und dem Orthesen-Gelenk bzw. dem Gelenk der Kniegelenk-Stütze bzw. deren Anbindungsstellen 4 um die Achse des Unterschenkels ermöglichen. Jedes dieser Gelenks-Elemente 9 besteht aus zwei Teilstücken, die gegeneinander bewegbar sind, nämlich ineinander bzw. aneinander entlang einer Kreissegmentbahn geführt sind, wobei die Kreissegmente beider Gelenks-Elemente 9 einem Kreis mit Mittelpunkt im zentralen Bereich des Beins zugehören. Vorzugsweise sind dabei die monozentrischen Gelenke 8 oberhalb der Gelenks-Elemente 9 für die Rotationsbewegung angeordnet.

Zusätzlich oder ebenfalls alternativ zu den beiden oben genannten Gelenken 8, 9 kann der Stützkorpus 1 mit einer Einrichtung zur Abstandsverstellung zwischen Orthesen-Gelenk bzw. Gelenk der Kniegelenk-Stütze und Schienbein-Schiene 2 an zumindest einem der beiden Äste 5, vorzugsweise natürlich an beiden Ästen 5, versehen sein. Ein Beispiel für eine derartige Einrichtung ist in der Zeichnungsfigur dargestellt und umfasst an jedem Ast 5 zwei Führungsbolzen 10, die zwischen dem Ende des seitlichen Astes 5 des Stützkorpus 1 und der darüber liegenden Struktur, hier dem Gelenks-Element 9, verlaufen und die beiden Teile 5, 9 linear zueinander führen. Die Einstellung des Abstandes zwischen dem Gelenks-Element 9 und dem seitlichen Ast 5 wird über eine Verstellschraube 11 bewerkstelligt, die mit gegenläufigen Gewinden in die beiden Teile 5, 9 eingreift. Die bzw. jede Einrichtung 10, 11 zur Abstandsverstellung kann unterhalb jedes vorgesehen Gelenks 8 bzw. Gelenk-Elementes 9 angeordnet sein.

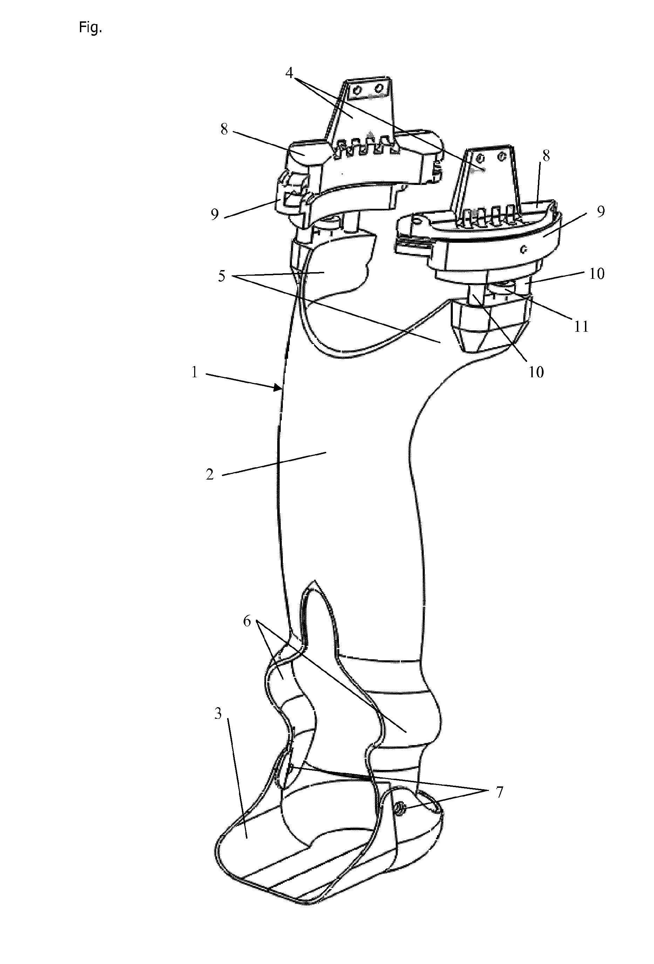

## Patentansprüche

1. Stützkorpus für Knie-Orthesen bzw. Kniegelenk-Stützen, in Form einer sich entlang des Unterschenkels erstreckenden und diesen teilweise umfassenden Schiene (2), die am oberen Ende mit einem Orthesen-Gelenk bzw. einem Gelenk der Kniegelenk-Stütze verbunden und am unteren Ende mit einem zumindest die Ferse aufnehmenden Fußteil (3) versehen ist, **dadurch gekennzeichnet, dass** am oberen Ende der Schiene (2) zwei seitlich des Beins angeordnete Anbindungsstellen (4) für die Knie-Orthese bzw. die Kniegelenk-Stützen vorgesehen sind, die in einen am Schienbein entlang des Beins nach unten verlaufenden Abschnitt übergehen, welcher seinerseits oberhalb des Knöchelbereiches wieder in zwei seitlich des Beins verlaufende Verbindungsteile (6) zum Fußteil (4) übergeht.

2. Stützkorpus nach Anspruch 1, **dadurch gekennzeichnet, dass** der Schienbein-Abschnitt (2) um das Bein herum gekrümmt oder geknickt ausgeführt ist, so dass das Bein über einen Teil seines Umfanges vom Abschnitt (2) abgedeckt ist.

3. Stützkorpus nach Anspruch 1 oder 2, **dadurch gekennzeichnet, dass** in den Verbindungsteilen (6) unterhalb der Knöchelhöhe jeweils ein Gelenk (7) zur schwenkbaren Verbindung zwischen Schienbein-Abschnitt (2) und Fußteil (3) vorgesehen ist.

4. Stützkorpus nach Anspruch 3, **dadurch gekennzeichnet, dass** die Achse der Gelenke (7) in den Verbindungsteilen (6) an der Fußaußenseite weiter in Richtung Ferse liegt als an der Fußinnenseite.

5. Stützkorpus nach einem der Ansprüche 1 bis 4, **dadurch gekennzeichnet, dass** das Orthesen-Gelenk bzw. das Gelenk der Kniegelenk-Stütze und die Schiene (2) über zwei monozentrische Gelenke (8) miteinander verbunden sind, deren Achsen im Wesentlichen in der Ebene der Beugebewegung des Kniegelenks orientiert sind.

6. Stützkorpus nach einem der Ansprüche 1 bis 5, **dadurch gekennzeichnet, dass** das Orthesen-Gelenk bzw. das Gelenk der Kniegelenk-Stütze und die Schiene (2) über zwei Gelenks-Elemente (9) miteinander verbunden sind, die eine Rotationsbewegung zwischen Stützkorpus und Orthesen-Gelenk bzw. Gelenk der Kniegelenk-Stütze um die Achse des Unterschenkels ermöglichen.

7. Stützkorpus nach Anspruch 6, **dadurch gekennzeichnet, dass** jedes Gelenks-Element (9) aus zwei ineinander bzw. aneinander entlang einer Kreissegmentbahn geführten Teilstücken besteht, wobei die Kreissegmente beider Gelenks-Elemente einem Kreis mit Mittelpunkt im zentralen Bereich des Beins zugehören.

8. Stützkorpus nach einem der Ansprüche 1 bis 7, **dadurch gekennzeichnet, dass** eine Einrichtung (10, 11) zur Abstandsverstellung zwischen Orthesen-Gelenk bzw. Gelenk der Kniegelenk-Stütze und Schienbein-Schiene (2) an zumindest einer der Anbindungsstellen, vorzugsweise an beiden Anbindungsstellen, vorgesehen ist.

9. Stützkorpus nach den Ansprüchen 5 bis 8, **dadurch gekennzeichnet, dass** monozentrischen Gelenke (8) oberhalb der Gelenks-Elemente (9) für die Rotationsbewegung angeordnet sind.

10. Stützkorpus nach den Ansprüchen 5 bis 9, **dadurch gekennzeichnet, dass** die bzw. jede Einrichtung (10, 11) zur Abstandsverstellung unterhalb jedes vorgesehen Gelenks (8, 9) bzw. Gelenk-Elementes angeordnet ist.
